Europäisches Patentamt

European Patent Office

Office européen des brevets

(19)

(11) Veröffentlichungsnummer: **0 145 666**
**B1**

## EUROPÄISCHE PATENTSCHRIFT

(12)

(45) Veröffentlichungstag der Patentschrift:
**22.06.88**

(51) Int. Cl.⁴: **A 61 B 17/58**

(21) Anmeldenummer: **84810592.0**

(22) Anmeldetag: **05.12.84**

(54) **Marknagel.**

(30) Priorität: **12.12.83 CH 6622/83**

(43) Veröffentlichungstag der Anmeldung:
**19.06.85 Patentblatt 85/25**

(45) Bekanntmachung des Hinweises auf die Patenterteilung:
**22.06.88 Patentblatt 88/25**

(84) Benannte Vertragsstaaten:
**AT BE CH DE FR GB LI SE**

(56) Entgegenhaltungen:
**GB - A - 537 235**
**GB - A - 1 593 440**

(73) Patentinhaber: **SYNTHES AG, Grabenstrasse 15,
CH-7002 Chur (CH)**

(72) Erfinder: **Mathys, Robert, Dr., Chrüzliacherstrasse 11,
CH-2544 Bettlach (CH)**
Erfinder: **Cotting, Anton, Dählenstrasse 66,
CH-2540 Grenchen (CH)**

(74) Vertreter: **Eder, Carl E. et al, Patentanwaltsbüro EDER
AG Münchensteinerstrasse 2, CH-4052 Basel (CH)**

## Beschreibung

Marknägel sind üblicherweise mit einem oder mehreren Längsschlitzen versehen, um zu gewährleisten, dass der Nagel die nötige Elastizität aufweist. Es gibt Nägel, bei denen das Gebiet an der Spitze nicht durch einen Schlitz unterbrochen ist, und solche, bei denen sich ein Längsschlitz von der Spitze bis in die Nähe des der Spitze abgewandten Endes erstreckt, wo der Umfang stets geschlossen ist, um zu gewährleisten, dass die für die Kraftübertragung beim Ein- und Ausschlagen des Nagels in diesem Teil benötigte Festigkeit vorhanden ist.

Die Erfahrung hat nun gezeigt, dass es wünschbar ist, dass ein Marknagel längs seiner ganzen Länge eine möglichst gleichmässige Elastizität aufweist, mit anderen Worten, dass die bei den bekannten Marknägeln am Schlitzende auftretenden Süannungsspitzen vermieden werden. Dieses Ziel wird nun mit dem Marknagel nach der vorliegenden Erfindung erreicht, der dadurch gekennzeichnet ist, dass sich der Längsschlitz über die ganze Länge des Nagels erstreckt und dass im Bereich des der Nagelspitze abgewandten Endes der Schlitz so ausgebildet ist, dass die einander benachbarten, in einer gemeinsamen Mantelfläche befindlichen, den Schlitz begrenzenden Randzonen so miteinander verhängt sind, dass ein Aufweiten des Nagels in diesem Bereich nicht möglich ist. Ein solcher Nagel weist also gegenüber einem üblichen Nagel gewisse Vorteile auf und besitzt trotzdem den Vorteil eines Nagels mit geschlossenem Ende, den Vorteil nämlich, dass sich konische Verbindungsstücke zum Ein- und Ausschlagen verwenden lassen. Um die beim Ein- und Ausschlagen des Nagels vom Einschlag- und Ausschlagwerkzeug auf den Nagel übertragenen Radialkraft-Komponenten möglichst klein zu halten, ist es von Vorteil, als Gewinde ein Trapez-Gewinde zu verwenden, dessen Flanken zur Längsachse des Marknagels symmetrisch geneigt sein sollen, wobei diese Neigung vorzugsweise ca. 85° beträgt.

Nachfolgend wird anhand der beiliegenden Zeichnung ein Ausführungsbeispiel der Erfindung beschrieben.

In der Zeichnung zeigt

die Figur 1 das der Spitze abgewandte Ende eines üblichen Marknagels,

die Figur 2 das der Spitze abgewandte Ende eines erfindungsgemässen Marknagels und

die Figur 3 einen Ausschnitt aus einem vergrösserten Axialschnitt durch das mit dem Gewinde versehene konische Ende eines Marknagels.

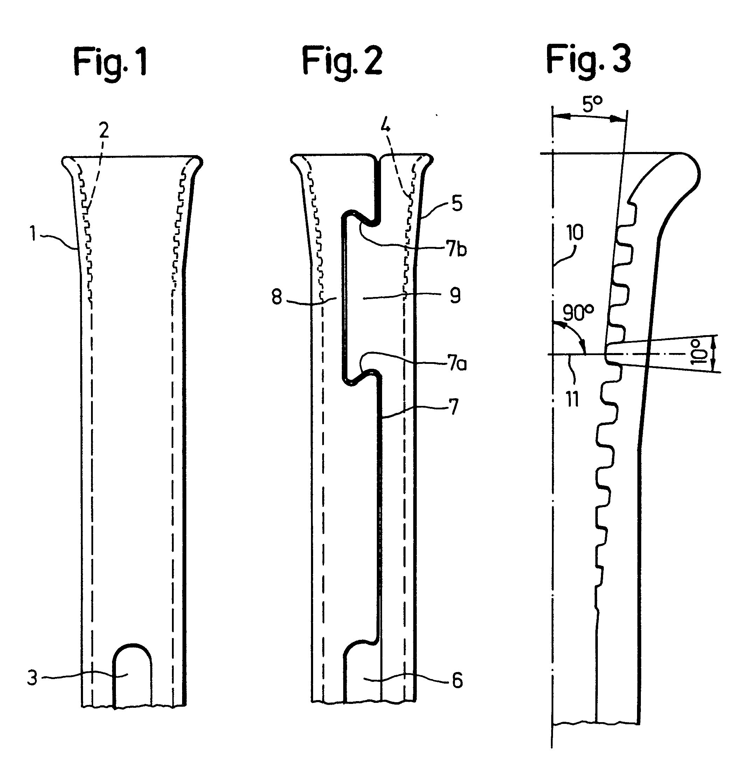

Der vorbekannte Marknagel, von welchem in der Fig. 1 das der Spitze abgewandte Ende dargestellt ist, ist im wesentlichen hülsen- oder hohlstabförmig. Er weist einen Mantel auf, welcher einen Hohlraum umgrenzt, der einen runden oder etwa dreiecken Querschnitt besitzt. Das dargestellte Nagelende weist eine konische Erweiterung 1 auf, die mit einem Innengewinde 2 versehen ist. Dieses Ende ist vollständig geschlossen. Ein mit 3 bezeichneter Längsschlitz erstreckt sich von der Spitze des Marknagels bis in die Nähe des konischen Teiles, so dass ein schlitzfreier Bereich von ca. 4–8 cm vorhanden ist. Am Ende dieses Schlitzes können nun Spannungskonzentrationen auftreten. Ein solcher Schlitz gewährleistet keine gleichmässige Elasiziät über die ganze Länge des Nagels.

Im Unterschied dazu erstreckt sich bei dem in Fig. 2 dargestellten erfindungsgemässen Marknagel, der an seinem der Spitze abgewandten Ende ebenfalls eine konische, mit einem Innengewinde 4 versehene Erweiterung 5 aufweist, der Schlitz 6 über die ganze Länge des Nagels, wobei allerdings im Endbereich, der dem geschlossenen Bereich des bekannten Nagels entspricht, der Schlitz sehr schmal ist, so dass die ihn begrenzenden Randzonen unter Umständen aneinander anstehen. Wesentlich ist nun, dass der Schlitz in diesem Bereich, wo er mit 7 bezeichnet ist, so ausgebildet ist, dass die einander benachbarten, in einer gemeinsamen Mantelfläche befindlichen, den Schlitz begrenzenden Randzonen 8 und 9 so miteinander verhängt sind, das ein Aufweiten des Nagels in diesem Bereich nicht möglich ist. Im speziellen Ausführungsbeispiel geschieht das dadurch, dass der Schlitz 7 im konischen Bereich eine Z- oder S-Biegung 7b und im zylindrischen Bereich zusätzlich noch eine dazu symmetrische Biegung 7a aufweist.

So weist eine Randzone 9 einen schwalbenschwanzartigen Fortsatz und die andere Randzone 8 eine schwalbenschwanzartige Einbuchtung auf.

Untersuchungen haben gezeigt, dass bei sorgfältiger Herstellung des Schlitzes, beispielsweise mittels eines Laserstrahles, der Zusammenhang der beiden einander benachbarten Randzonen 8 und 9 so gut ist, dass die in der Praxis bewährten konischen Ein- und Ausschlaginstrumente in den konischen Abschnitt des Marknagels eingeschraubt werden können, ohne dass sich dieses Ende aufweitet, so dass sich dieser Nagel genau gleich handhaben lässt wie der an sich bekannte Nagel.

Bei den an sich bekannten Nägeln weist das Gewinde einen dreieckigen Spitzenprofil-Querschnitt auf. Da bei einem solchen Gewinde beim Ein- und Ausschlagen die radiale Kraftkomponente verhältnismässig gross ist, ist es von Vorteil, wenn man beim erfindungsgemässen Nagel ein Trapez-Gewinde verwendet, beispielsweise von der Art, wie es in der Figur 3 dargestellt ist. Bei einem Konuswinkel von 5° werden die Flanken des trapezförmigen Querschnitts zweckmässigerweise so ausgebildet, dass sie mit einer auf der Nagelachse 10 senkrecht stehenden Linie 11 einen Winkel von ca. 5° bilden, mit andern Worten so, dass die beiden Flanken miteinander einen Winkel von ca. 10° einschliessen und dass ihre Symmetrielinie 10 senkrecht zur Nagelachse 11 verläuft. Mit dieser Massnahme können die radialen Kräfte, die auf die Z- oder S-Biegung 7b bzw. 7a einwirken, sehr klein gehalten werden, so dass die Gefahr vermindert wird, dass die Stossstelle beschädigt werden könnte.

## Patentansprüche

1. Marknagel mit Längsschlitz (6), dadurch gekennzeichnet, dass sich der Längsschlitz über die ganze Länge des Nagels erstreckt und dass im Bereich des der Nagelspitze abgewandten Endes der Schlitz (7) so ausgebildet ist, dass die einander benachbarten, in einer gemeinsamen Mantelfläche befindlichen, den Schlitz (7) begrenzenden Randzonen (8, 9) so miteinander verhängt sind, dass ein Aufweiten des Nagels in diesem Bereich nicht möglich ist.

2. Marknagel nach Anspruch 1, dadurch gekennzeichnet, dass der Schlitz (7) im Bereich des der Nagelspitze abgewandten Endes mindestens eine Z- oder S-Biegung (7b), vorzugsweise aber zusätzlich noch eine dazu symmetrische Biegung (7a) aufweist.

3. Marknagel nach einem der Ansprüche 1 oder 2, der im wesentlichen hülsen- oder hohlstabförmig ist und einen Mantel aufweist, welcher beispielsweise einen im Querschnitt, abgesehen vom Schlitz (6), geschlossenen Hohlraum begrenzt, dadurch gekennzeichnet, dass die einander zugewandten, den Schlitz (6) begrenzenden Randzonen (8, 9) des Mantels in einem Längsabschnitt des Mantels miteinander verhängt sind.

4. Marknagel nach einem der Ansprüche 1 bis 3, dessen der Nagelspitze abgewandter Endabschnitt (5) sich von der Nagelspitze weg konisch erweitert und mit einem Innengewinde (4) versehen ist, dadurch gekennzeichnet, dass sich mindestens ein Teilabschnitt der miteinander verhängten Randzonen (8, 9) im Bereich der Erweiterung befindet, wobei der Schlitz beispielsweise im Bereich der Erweiterung (5) einen Z- oder S-förmig verlaufenden Abschnitt (7b) aufweist.

5. Marknagel nach Anspruch 4, dadurch gekennzeichnet, dass das Innengewinde ein Trapez-Gewinde ist.

6. Marknagel nach Anspruch 5, dadurch gekennzeichnet, dass im Axialschnitt die Symmetrielinie zweier Flanken jedes Trapezes die Achse des Marknagels senkrecht schneidet.

7. Marknagel nach Anspruch 6, dadurch gekennzeichnet, dass die beiden Flanken jedes Trapezes gegeneinander um 10° geneigt sind.

8. Marknagel nach einem der Ansprüche 1 bis 7, dadurch gekennzeichnet, dass Konuswinkel der konischen Erweiterung 5° beträgt.

9. Marknagel nach einem der Ansprüche 1 bis 8, dessen mittlerer Abschnitt profilstabförmig ist und beispielsweise, abgesehen vom Schlitz (6), mindestens annähernd einen Hohlzylinder oder ein Dreikanten-Prisma mit verrundeten Übergängen zwischen den benachbarten Seitenflächen bildet, dadurch gekennzeichnet, dass sich mindestens ein Teilabschnitt der miteinander verhängten Randzonen (8, 9) im Bereich des profilstabförmigen Abschnittes befindet, wobei der Schlitz vorzugsweise im profilstabförmigen Abschnitt mindestens einen Z- oder S-förmig verlaufenden Abschnitt (7a) aufweist.

10. Marknagel nach einem der Ansprüche 1 bis 9, dadurch gekennzeichnet, dass der Schlitz (6) in demjenigen Längsabschnitt des Marknagels, in dem die Randzonen (8, 9) miteinander verhängt sind, einen Abschnitt (7) aufweist, der schmäler ist, als der auf der der Nagelspitze zugewandten Seite an ihn anschliessende Abschnitt, wobei sich der breitere Schlitzabschnitt vorzugsweise vom schmäleren Schlitzabschnitt (7) bis mindestens annähernd zur Nagelspitze erstreckt.

11. Marknagel nach einem der Ansprüche 1 bis 10, dadurch gekennzeichnet, dass die eine Randzone (9) in demjenigen Längsabschnitt des Marknagels, in welchem die Randzonen miteinander verhängt sind, einen Vorsprung aufweist, der sich von seiner Wurzel weg zu seinem dieser abgewandten Rand hin erweitert und beispielsweise ungefähr schwalbenschwanzförmig ausgebildet ist, und dass die andere Randzone (8) eine Ausnehmung aufweist, in die der Vorsprung eingreift, wobei der Vorsprung vorzugsweise satt oder mit höchstens geringem Spiel in die Ausnehmung hineinpasst, wobei diejenigen Abschnitte (7a, 7b) des Schlitzes, die die beiden seitlichen Flanken des Vorsprunges und die an diese anschliessenden Schlitzbereiche bilden, beispielsweise Z-förmig abgewinkelt oder S-förmig gebogen sind und wobei derjenige Längsabschnitt des Schlitzes (6), bei dem die Randzonen nicht miteinander verhängt sind, vorzugsweise entlang einer Mantellinie verläuft.

12. Marknagel nach Anspruch 11, dadurch gekennzeichnet, dass sich der Vorsprung zum Teil im Bereich einer sich in von der Nagelspitze weggerichteten Richtung erweiternden, beispielsweise ungefähr konischen, Erweiterung (5) und zum Teil in einem im allgemeinen profilstabförmigen, beispielsweise mindestens annähernd zylindrischen, Bereich des Marknagels befindet.

## Revendications

1. Clou intramédullaire à fente longitudinale (6), caractérisé en ce que la fente longitudinale s'étend sur toute la longueur du clou et en ce que, dans la zone de l'extrémité éloignée de la pointe du clou, la fente (7) est agencée de façon telle que les zones marginales (8, 9), voisines l'une de l'autre, qui limitent la fente (7) et qui sont situées sur une surface latérale commune, s'interpénètrent de telle manière qu'un élargissement du clou dans cette zone ne soit pas possible.

2. Clou intramédullaire selon la revendication 1, caractérisé en ce que, dans la zone de l'extrémité -éloignée de la pointe du clou, la fente (7) possède au moins une courbure (7b) en Z ou en S, mais de préférence en plus une courbure (7a) symétrique de la première.

3. Clou intramédullaire selon l'une des revendications 1 et 2, qui a essentiellement la forme d'un manchon ou d'une tige creuse et possède une surface latérale qui limite par exemple une cavité de section fermée, à l'exception de la fente (6), caractérisé en ce que les zones marginales (8, 9) de la surface latérale, qui sont tournées l'une vers l'autre et qui limitent la fente (6), s'interpénètrent dans une partie longitudinale de la surface latérale.

4. Clou intramédullaire selon l'une des revendications 1 à 3, dont la partie terminale (5) éloignée de la pointe du clou s'élargit coniquement en s'écartant de la pointe du clou et est munie d'un filetage intérieur (4), caractérisé en ce qu'au moins une partie des zones marginales s'interpénétrant (8, 9) se trouve dans la région de l'élargissement, la fente présentant, par exemple dans la région de l'élargissement (5), une partie (7b) de forme en Z ou en S.

5. Clou intramédullaire selon la revendication 4, caractérisé en ce que le filetage intérieur est un filetage en trapèze.

6. Clou intramédullaire selon la revendication 5, caractérisé en ce que, en coupe axiale, l'axe de symétrie des deux flancs de chaque trapèze coupe à angle droit l'axe du clou intramédullaire.

7. Clou intramédullaire selon la revendication 6, caractérisé en ce que les deux flancs de chaque trapèze sont inclinés de 10° l'un par rapport à l'autre.

8. Clou intramédullaire selon l'une des revendications 1 à 7, caractérisé en ce que l'angle au sommet de l'élargissement conique est égal à 5°.

9. Clou intramédullaire selon l'une des revendications 1 à 8, dont la partie médiane a la forme d'une tige profilée et constitue par exemple, à l'exception de la fente (6), au moins approximativement un cylindre creux ou un prisme à trois faces et à angles arrondis entre les faces latérales voisines, caractérisé en ce qu'au moins une partie des zones marginales s'interpénétrant (8, 9) se trouve dans la région de la partie en forme de tige profilée, la fente possédant au moins une partie (7a) en Z ou en S, de préférence dans la partie en forme de tige profilée.

10. Clou intramédullaire selon l'une des revendications 1 à 9, caractérisé en ce que, dans celle des parties longitudinales du clou intramédullaire dans laquelle les zones marginales (8, 9) s'interpénètrent, la fente (6) possède une partie (7) qui est plus étroite que la partie qui s'y raccorde sur le côté tourné vers la pointe du clou, la partie plus large de la fente s'étendant de préférence de la partie plus étroite (7) de la fente à, au moins approximativement, la pointe du clou.

11. Clou intramédullaire selon l'une des revendications 1 à 10, caractérisé en ce que, dans celle des parties longitudinales du clou intramédullaire dans laquelle les zones marginales s'interpénètrent, l'une des zones marginales (9) possède une saillie qui s'élargit de sa racine à son bord éloigné de cette dernière et a par exemple à peu près une forme en queue-d'aronde, et en ce que l'autre zone marginale (8) possède un évidement dans lequel est engagée la saillie, la saillie s'adaptant de préférence juste ou tout au plus avec un faible jeu dans l'évidement, celles des parties (7a, 7b) de la fente qui forment les deux flancs latéraux de la saillie et les parties de la fente se raccordant à ceux-ci, ayant par exemple une forme repliée en Z ou courbée en S et celle des parties longitudinales de la fente (6) dans laquelle les zones marginales ne s'interpénètrent pas s'étendant de préférence le long d'une génératrice.

12. Clou intramédullaire selon la revendication 11, caractérisé en ce que la saillie se trouve en partie dans la région d'un élargissement (5), par exemple à peu près conique, qui s'élargit dans le sens opposé à la pointe du clou et en partie dans une région du clou intramédullaire qui a la forme générale d'une tige profilée, par exemple au moins approximativement cylindrique.

**Claims**

1. Medullary nail with longitudinal slot (6), characterised thereby, that the longitudinal slot extends over the entire length of the nail and that the slot (7) is so formed in the region of the end remote from the nail tip that the mutually adjacent edge zones (8, 9), which are disposed in a common envelope surface and bound the slot (7), are each so interlocked with the other that a splaying of the nail is not possible in this region.

2. Medullary nail according to claim 1, characterised thereby, that the slot (7) displays at least one Z-bend or S-bend (7b), preferably however still additionally a bend (7a) symmetrical thereto, in the region of the end remote from the nail tip.

3. Medullary nail according to one of the claims 1 or 2, which is substantially in the shape of a sleeve or of a hollow rod and displays an envelope which bounds a hollow space closed in cross-section apart from the slot (6), characterised thereby, that the mutually adjacent edge zones (8, 9) of the envelope, which bound the slot (7), are each interlocked with the other in a longitudinal portion of the envelope.

4. Medullary nail according to one of the claims 1 to 3, the end portion (5) of which remote from the nail tip enlarges conically away from the nail tip and is provided with an internal thread (4), characterised thereby, that at least a partial portion of the mutually interlocked edge zones (8, 9) is disposed in the region of the enlargement, wherein the slot, for example in the region of the enlargement (5), displays a portion (7b) extending in Z-shape or in S-shape.

5. Medullary nail according to claim 4, characterised thereby, that the internal thread is a trapezoidal thread.

6. Medullary nail according to claim 5, characterised thereby, that the line of symmetry of two flanks of each trapezium cuts the axis of the medullary nail perpendicularly in the axial section.

7. Medullary nail according to claim 6, characterised thereby, that both the flanks of each trapezium are each inclined relative to the other through 10°.

8. Medullary nail according to one of the claims 1 to 7, characterised thereby, that the cone angle of the conical enlargement amounts to 5°.

9. Medullary nail according to one of the claims 1 to 8, the middle portion of which is in the shape of a profiled rod and, apart from the slot (6), for example at least approximately forms a hollow cylinder or a triangular prism with rounded transitions between the neighbouring side surfaces, characterised thereby, that at least a partial portion of the mutually interlocked edge zones (8, 9) is disposed in the region of the portion in the shape of a profiled rod, displays a portion (7a) extending in Z-shape or in S-shape.

10. Medullary nail according to one of the claims 1 to 9, characterised thereby, that the slot (6) in that longitudinal portion of the medullary nail, in which the edge zones (8, 9) are each interlocked with the other, displays a portion (7), which is narrower than the portion adjoining it at the side facing the nail tip, wherein the wider slot portion preferably extends from the narrower slot portion (7) at least nearly to the nail tip.

11. Medullary nail according to one of the claims 1 to 10, characterised thereby, that the one edge zone (9) in that longitudinal portion of the medullary nail, in which the edge zones are each interlocked with the other, displays a projection which enlarges away from its root towards its edge remote therefrom and is for example constructed approximately in dovetail shape, and that the other edge zone (8) displays a recess, into which the projection engages, wherein the projection fits into the recess preferably flush or with at most little play, wherein those portions (7a, 7b) of the slot, which form both the lateral flanks of the projection and the slot regions adjoining thereat, are for example angled in Z-shape or bent in S-shape and wherein that longitudinal portion of the slot (6), in which the edge zones are not each interlocked with the other, preferably extends along an envelope line.

12. Medullary nail according to claim 11, characterised thereby, that the projection is disposed in part in the region of an enlargement (5), which enlarges in a direction directed away from the nail tip and is for example approximately conical, and in part in a region, which is generally in the shape of a profiled rod and for example at least approximately cylindrical, of the medullary nail.

# Fig. 1

2

1

3

# Fig. 2

4

5

7b

8

9

7a

7

6

# Fig. 3

5°

10

90°

11

10°